Europäisches Patentamt

**European Patent Office**

Office européen des brevets

(11) Veröffentlichungsnummer: **0 075 736**
**B1**

(12) **EUROPÄISCHE PATENTSCHRIFT**

(45) Veröffentlichungstag der Patentschrift:
27.12.85

(51) Int. Cl.⁴: **A 61 M 29/02**

(21) Anmeldenummer: **82107921.7**

(22) Anmeldetag: **28.08.82**

(54) **Dilatationsvorrichtung.**

(30) Priorität: **29.09.81 DE 3138620**

(43) Veröffentlichungstag der Anmeldung:
**06.04.83 Patentblatt 83/14**

(45) Bekanntmachung des Hinweises auf die Patenterteilung:
**27.12.85 Patentblatt 85/52**

(84) Benannte Vertragsstaaten:
**AT BE CH DE FR GB IT LI LU NL SE**

(56) Entgegenhaltungen:
**WO - A - 80/02366**
**DE - A - 2 049 959**
**DE - A - 2 320 014**
**DE - C - 811 717**
**GB - A - 1 097 882**
**US - A - 4 273 128**

**MEDICAL PROGRESS TECHNOLOGY Band 8, Nr. 1,
1980, Berlin J.-C. FARCOT et al. "Pump system for
diastolic synchronized coronary sinus retroperfusion",
Seiten 29 bis 37**

(73) Patentinhaber: **Kuhl, Adolf, Dr. med. Ing.-grad.,
Finkenhof 3b, D-3000 Hannover 61 (DE)**

(72) Erfinder: **Kuhl, Adolf, Dr. med. Ing.-grad., Finkenhof 3b,
D-3000 Hannover 61 (DE)**

(74) Vertreter: **Selting, Günther, Dipl.-Ing. et al,
Deichmannhaus am Hauptbahnhof, D-5000 Köln 1 (DE)**

## Beschreibung

Die Erfindung betrifft eine Dilatationsvorrichtung zum Aufweiten oder Verschließen von Gefäßen und anderen Körperhohlräumen, mit einem an eine Druckquelle anschließbaren Katheter, der ein aufweitbares Dilatationselement aufweist.

Aus GB-A-1 097 882, US-A-4 273 128 und DE-C-811 717 ist es bekannt, mit Hilfe von aufblasbaren Kathetern Gefäße und andere Hohlräume im menschlichen Körper zu öffnen oder zu erweitern bzw. zu verschließen. Hierbei werden Dilatationskatheter benutzt, die an ihrem Ende ein aufweitbares Dilatationselement aufweisen. Bei der Verlegung des Katheters wird das Dilatationselement an die aufzuweitende Stelle gebracht und anschließend aufgepumpt. Nachteilig ist hierbei, daß für die Zeit des Aufpumpens das betreffende Gefäß bzw. der Hohlraum durch das Dilatationselement blockiert wird, so daß die Versorgung der nachgeordneten Organe, z. B. mit Blut für die Dauer der Behandlung unterbrochen wird. DE-C-811 717 beschreibt außerdem bei einem Dilatationskatheter die Anwendung eines Überdruckventils zur Begrenzung des Druckes im Innern des als aufweitbares Dilatationselement dienenden Schlauchs. Dadurch, daß ein Druck gewählt wird, der über dem Ansprechdruck des Überdruckventils liegt, wird die Möglichkeit geschaffen, den Schlauch periodisch ausdehnen und schrumpfen zu lassen, um auf diese Weise das zu dehnende Gefäß periodisch aufzuweiten.

Der Erfindung liegt die Aufgabe zugrunde, eine Dilatationsvorrichtung der eingangs genannten Art zu schaffen, die ein abwechselndes Ausdehnen und Zusammenziehen des Dilatationselementes im Rhythmus der Herzfrequenz ermöglicht, um das betreffende Gefäß bzw. den Körperhohlraum zwischen kurzzeitigen Dilatationsphasen für den Flüssigkeitsdurchlauf freizugeben.

Zur Lösung dieser Aufgabe ist erfindungsgemäß vorgesehen, daß die Druckquelle einen pulsierenden Druck liefert, dessen unterer Druckwert als Betriebsdruck mit der Dehnungscharakteristik des Dilatationselementes derart abgestimmt ist, daß er im Bereich des steilen Druckanstiegs bei nahezu konstantem Volumen dicht unterhalb des steilen Volumenanstiegs liegt, und dessen oberer Druckwert im Bereich des steilen Volumenanstiegs liegt, wo eine Änderung des Druckes p eine definierte Volumenänderung v zur Folge hat, und daß das Dilatationselement derart ausgebildet ist, daß sein Volumen oberhalb des Betriebsdruckes Druckpulsen mit einer Folgefrequenz von 0,5 bis 3 Hz folgt.

Die Erfindung geht von dem Gedanken aus, daß die Aufweitung eines Dilatationselementes, das Bestandteil eines Katheters ist, gegenüber der Druckbeaufschlagung durch die Druckquelle normalerweise eine Verzögerung oder Phasenverschiebung zu den Druckimpulsen der Druckquelle hat, wenn die Druckquelle jeweils zwischen zwei Druckimpulsen ganz oder nahezu drucklos würde. Dies ist auf den Strömungswiderstand des Katheters und auf die Druck/Volumen-Charakteristik des ballonartigen Dilatationselementes zurückzuführen. Wird das Dilatationselement, ausgehend vom drucklosen Zustand, aufgepumpt, dann erhöht sich zunächst der Druck im Dilatationselement, ohne daß eine wesentliche Volumenvergrößerung erfolgt. Erst von einem bestimmten Druckwert an, der als Kniepunkt bezeichnet werden kann, ergibt sich ein wesentlicher Volumengradient, d. h. das Volumen des Dilatationselementes steigt mit zunehmendem Druck stark an. Durch die Erfindung wird der Betriebsdruck dicht unter den Kniepunkt der Druck/Volumen-Charakteristik gelegt, so daß auch zwischen den Druckimpulsen ein gewisser Vordruck im Dilatationselement aufrechterhalten wird. Die für die Volumenvergrößerung des Dilatationselementes erforderliche Menge an Druckfluid ist, ausgehend von diesem Vordruck, relativ klein. Auf diese Weise ist es möglich, mit geringen Strömungsmengen im Katheter große Volumenänderungen des Dilatationselementes hervorzurufen, so daß das Dilatationselement im Rhythmus der Herzfrequenz aufgepumpt und zusammengezogen werden kann. Dabei ist es nicht erforderlich, bei jedem Pulsschlag einen aus Aufpumpen und Zusammenziehen des Dilatationselementes bestehenden Zyklus durchzuführen, sondern das Dilatationselement kann auch über einen oder mehrere Pulsschläge hinweg aufgepumpt bzw. zusammengezogen bleiben. Wichtig ist nur, daß der Vorgang des Aufpumpens und Zusammenziehens relativ schnell und möglichst trägheitsarm erfolgt.

In vorteilhafter Weiterbildung der Erfindung ist vorgesehen, daß der Katheter zwischen dem Druckquellenanschluß und dem Dilatationselement aus einem Schlauch besteht, dessen radiale Dehnungsfähigkeit wesentlich kleiner ist als die des Dilatationselementes und daß der Schlauchdurchmesser sich in einem Abstand von dem Dilatationselement zur Druckquelle hin vergrößert. Diese Erweiterung des Innenquerschnitts des Schlauches hat den Zweck, den Strömungswiderstand des Katheters zu reduzieren. Damit wird die obere Grenzfrequenz zur Übertragung von Druckpulsen zu höheren Frequenzen hin verschoben. Vorzugsweise liegt die obere Grenzfrequenz der Übertragung von Druckimpulsen von der Druckquelle zum Dilatationselement bei etwa 100 Hz. Hierdurch können den Druckimpulsen höherfrequente Druckschwankungen überlagert werden, die beispielsweise dreieckförmigen, sägezahnförmigen oder sinusförmigen zeitlichen Verlauf haben. Durch derartige Vibrationen, die einem Druckimpuls überlagert sind, wird die Gefäßdilatation verbessert.

Um während des Vorschiebens des Katheters an den Dilatationsort eine Druckmessung zu ermöglichen, ist gemäß einer vorteilhaften Ausgestaltung der Erfindung vorgesehen, daß der Katheter einen Druckmeßkanal aufweist, dessen vorderes Ende über das Dilatationselement hinaus vorsteht. Der vorstehende Teil ist weich ausgebildet, so daß Verletzungen von Körperhohlräumen und Gefäßen vermieden werden.

Vorzugsweise erlaubt die Dehnungscharakteristik des Dilatationselementes eine reproduzierbare Volumenänderung des Dilatationselementes durch den eingestellten Druck.

Gemäß einer vorteilhaften Weiterbildung der Erfindung ist vorgesehen, daß die Druckquelle derart ausgebildet ist, daß der Druck in dem Dilatationselement bei jedem Druckimpuls zunächst langsam ansteigt bis das Dilatationselement an der Wand eines Körperhohlraums anliegt, was an dem diastolisch nicht mehr abfallenden Druck in einem an die Druckquelle angeschlossenen Druckbehälter erkennbar ist.

Zweckmäßigerweise wird der in dem Druckbehälter herrschende Druck zur Festlegung des bei der Dilatation einzustellenden Druckes im Dilatationselement herangezogen.

Im folgenden wird unter Bezugnahme auf die Zeichnungen ein Ausführungsbeispiel der Erfindung näher erläutert. Es zeigt

Fig. 1 eine Querschnittsdarstellung eines Dilatationskatheters, und

Fig. 2 das Druckvolumen-Diagramm des Dilatationselementes, bei dem Katheter nach Fig. 1.

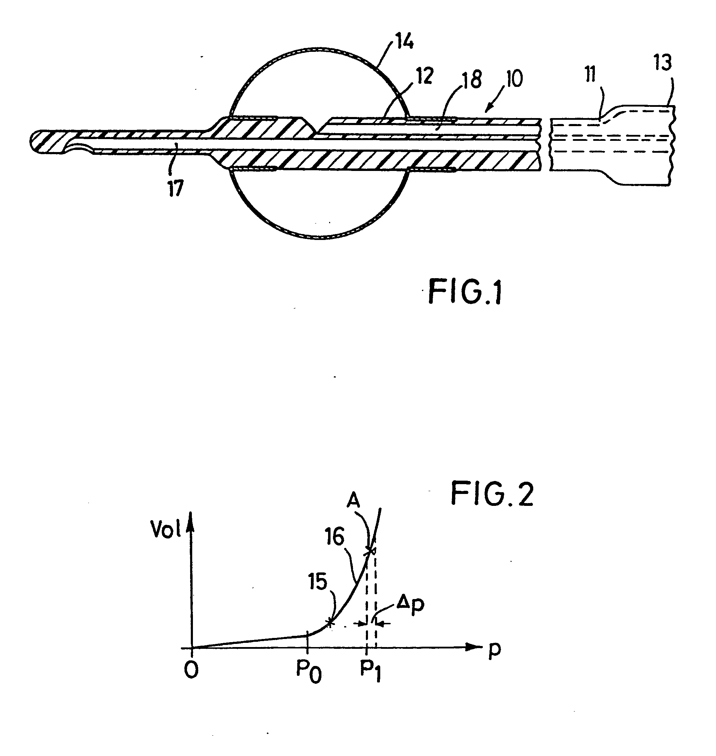

Der in Fig. 1 dargestellte Dilatationskatheter 10 besteht aus einem Schlauch 11, dessen patientenseitiger Abschnitt 12 auf einer Länge 10 bis 15 cm einen kleineren Durchmesser hat als die restliche Schlauchlänge 13. Der Schlauch 11 weist zwei längslaufende Kanäle 17, 18 auf. Das rückwärtige Ende des Kanals 18 wird an eine Druckquelle angeschlossen. An dem offenen vorderen Ende des Schlauchabschnitts 12 ist das Dilatationselement 14, z. B. durch Kleben, befestigt. Das Dilatationselement 14 besteht aus einem dehnungsfähigen Ballon oder Schlauchstück, das den Abschnitt 12 des Katheters 10 abdichtend umschließt und in das der Kanal 18 einmündet, so daß es durch den Druck im Kanal 18 ballonartig aufgepumpt werden kann. Die radiale Dehnungsfähigkeit des Dilatationselementes 14 ist wesentlich größer als diejenige des Schlauches 11. Wenn in dem Dilatationskatheter 10 durch ein eingeführtes Gas oder eine Flüssigkeit ein Druck erzeugt wird, behält der Schlauch 11 seinen Durchmesser im wesentlichen bei, während das Dilatationselement 14 aufgeweitet wird.

In Fig. 2 ist das Druck/Volumen-Diagramm des Dilatationsteils 14 dargestellt, wobei auf der Abszisse der Überdruck p im Innern des Dilatationselementes 14 gegenüber dem Außendruck dargestellt ist. Bei von dem Wert 0 aus ansteigendem Druck p vergrößert sich zunächst das Volumen des Dilatationselementes 14 nur sehr gering, bis zu einem Kniepunkt 15. Bei weiter ansteigendem Druck p findet eine starke Volumenänderung statt, was sich in dem Diagramm dadurch äußert, daß die Volumenkurve mit zunehmendem Druck ansteigt.

Die an den Katheter 10 angeschlossene Druckquelle ist so ausgebildet, daß sie Druckimpulse erzeugt, deren unterer Druckwert einem Arbeitsdruck $p_0$ entspricht, der dicht unterhalb des Kniepunktes 15 liegt, während der obere Druckwert $p_1$ auf dem ansteigenden Ast 16 des Druck/Volumen-Diagramms liegt. Den Druckimpulsen, bei denen der Druck den größeren Wert $p_1$ annimmt, können hochfrequente Impulsschwingungen mit einer Frequenz von ca. 100 Hz überlagert werden, wodurch sie periodisch ein Druckanstieg $\Delta p$ gegenüber dem Wert $p_1$ ergibt. Der Arbeitspunkt A liegt in der Mitte des Intervalls $\Delta p$.

Der Katheter 10 weist ferner einen Druckmeßkanal 17 auf, der über das Dilatationselement 14 nach vorne vorsteht. Der Druckmeßkanal 17, der an seinem vorderen Ende seitlich offen ist, dient der Druckmessung während des Vorschiebens des Katheters 10 an den Dilatationsort. Er erstreckt sich über die gesamte Länge des Katheters 10. Der Katheter 10 ist also doppellumig ausgeführt, wobei der Kanal 18 der Druckbeaufschlagung des Dilatationselementes 14 und der Kanal 17 als Druckmeßkanal dient.

## Patentansprüche

1. Dilatationsvorrichtung zum Aufweiten oder Verschließen von Gefäßen und anderen Körperhohlräumen, mit einem an eine Druckquelle anschließbaren Katheter (10), der ein aufweitbares Dilatationselement (14) aufweist, dadurch gekennzeichnet, daß die Druckquelle einen pulsierenden Druck liefert, dessen unterer Druckwert als Betriebsdruck ($p_0$) mit der Dehnungscharakteristik des Dilatationselementes (14) derart abgestimmt ist, daß er im Bereich des steilen Druckanstiegs bei nahezu konstantem Volumen dicht unterhalb des steilen Volumenanstiegs (16) liegt, und dessen oberer Druckwert ($p_1$) im Bereich des steilen Volumenanstiegs (16) liegt, wo eine Änderung des Druckes p eine definierte Volumenänderung v zur Folge hat, und daß das Dilatationselement (14) derart ausgebildet ist, daß sein Volumen oberhalb des Betriebsdruckes ($p_0$) Druckpulsen mit einer Folgefrequenz von 0,5 bis 3 Hz folgt.

2. Dilatationsvorrichtung nach Anspruch 1, dadurch gekennzeichnet, daß der Katheter (10) zwischen dem Druckquellenanschluß und dem Dilatationselement (14) aus einem Schlauch (11) besteht, dessen radiale Dehnungsfähigkeit wesentlich kleiner ist als die des Dilatationselementes (14) und daß der Schlauchdurchmesser sich in einem Abstand von dem Dilatationselement (14) zur Druckquelle hin vergrößert.

3. Dilatationsvorrichtung nach Anspruch 1 oder 2, dadurch gekennzeichnet, daß die obere Grenzfrequenz der Übertragung von Druckimpulsen von der Druckquelle zum Dilatationselement (14) etwa 100 Hz beträgt.

4. Dilatationsvorrichtung nach einem der vorhergehenden Ansprüche, dadurch gekennzeichnet, daß der Katheter (10) einen Druckmeßkanal (17) aufweist, dessen vorderes Ende über das Dilatationselement (14) hinaus vorsteht.

5. Dilatationsvorrichtung nach einem der vorhergehenden Ansprüche, dadurch gekennzeichnet, daß die Dehnungscharakteristik des Dilatationselementes (14) eine reproduzierbare Volumenänderung des Dilatationselementes durch den eingestellten Druck erlaubt.

6. Dilatationsvorrichtung nach einem der Ansprüche 1 bis 4, dadurch gekennzeichnet, daß die Druckquelle derart ausgebildet ist, daß der Druck in dem Dilatationselement (14) bei jedem Druckimpuls zunächst langsam ansteigt bis das Dilatationselement (14) an der Wand eines Körperhohlraums anliegt, was an dem diastolisch nicht mehr abfallenden Druck in einem an die Druckquelle angeschlossenen Druckbehälter erkennbar ist.

7. Dilatationsvorrichtung nach Anpruch 6, dadurch gekennzeichnet, daß der in dem Druckbehälter herrschende Druck zur Festlegung des bei der Dilatation einzustellenden Druckes im Dilatationselement (14) herangezogen wird.

## Claims

1. Dilatation means for enlarging or closing vessels and other body cavities, comprising a catheter (10) connectible to a pressure source and having an expandable dilatation element (14), characterized in that the pressure source supplies a pulsating pressure whose lower value is so harmonized as an operating pressure ($p_0$) with the dilatation characteristics of the dilatation element (14) that, within the range of the steep pressure rise, it is, with a nearly constant volume, closely beneath the steep volume rise (16), and whose upper pressure value ($p_1$) is within the range of the steep volume rise (16) where a change of the pressure p results in a defined volume change v, and that the dilatation element (14) is so designed that its volume above the operational pressure (po) follows pressure pulses of a sequence frequency from 0.5 to 3 Hz.

2. Dilatation means according to claim 1, characterized in that the catheter (10) between the pressure source terminal and the dilatation element (14) consists of a hose (11) whose radial dilatability is substantially inferior to that of the dilatation element (14), and that the hose diameter is increased towards the pressure source at a distance from the dilatation element (14).

3. Dilatation means according to claim 1 or 2, characterized in that the upper limit frequency of the transmission of pressure pulses from the pressure source to the dilatation element (14) is about 100 Hz.

4. Dilatation means according to one of the preceding claims, characterized in that the catheter (10) includes a pressure measuring channel (17) whose front end projects beyond the dilatation element (14).

5. Dilatation means according to one of the preceding claims, characterized in that the dilatation characteristics of the dilatation element (14) permit by the adjusted pressure a reproducible volume change of the dilatation element.

6. Dilatation means according to one of claim 1 to 4, characterized in that the pressure source is so designed that with each pressure pulse, the pressure in the dilatation element first slowly rises until the dilatation element (14) adjoins the wall of a body cavity which is detectable by the fact that the pressure in a pressure vessel connected to the pressure source does not further decrease diastolically.

7. Dilatation means according to claim 6, characterized in that the pressure existing in the pressure vessel is used to determine the pressure to be set with the dilatation in the dilatation element (14).

## Revendications

1. Dispositif dilatateur destiné à élargir ou à obturer des vaisseaux ou autres cavités corporelles comportant un cathéter (10) pouvant être raccordé à une source de pression, cathéter qui présente un élément dilatateur (14) pouvant être élargi, caractérisé par le fait que la source de pression délivre une pression pulsatoire dont la valeur inférieure, servant de pression de service ($p_0$) est adaptée à la courbe caractéristique de dilatation de l'élément dilatateur (14) de telle manière que cette pression se situe dans la zone d'élévation rapide de pression, à volume pratiquement constant, directement en dessous de tronçon (16) à augmentation rapide du volume, et dont la valeur de pression supérieure ($p_1$) se situe dans la zone du tronçon (16) à augmentation rapide du volume, à un endroit où une modification de la pression p a pour conséquence une modification définie du volume v, et par le fait que l'élément dilatateur (14) est conformé de telle manière qu'au-dessus de la pression de service ($p_0$) son volume suive les impulsions de pression à la freéquence séquentielle de 0,5 à 3 Hz.

2. Dispositif dilatateur selon la revendication 1, caractérisé par le fait que la cathéter (10) entre le raccord à la source de pression et l'élément dilatateur (14) est constitué par un tuyau (11) dont la capacité de dilatation radiale est sensiblement plus faible que celle de l'élément dilatateur (14) et par le fait que le diamètre du tuyau s'agrandit à une certaine distance de l'élément dilatateur (14) en direction de la source de pression.

3. Dispositif dilatateur selon les revendications 1 ou 2, caractérisé par le fait que la fréquence limite supérieure de la transmission d'impulsions de pression de la source de pression vers l'élément dilatateur (14) est d'environ 100 Hz.

4. Dispositif dilatateur selon l'une des revendications précédentes, caractérisé par le fait que le cathéter (10) présente un canal manométrique (17) dont l'extrémité avant dépasse à l'extérieur de l'élément dilatateur (14).

5. Dispositif dilatateur selon l'une des revendications précédentes, caractérisé par le fait que la courbe caractéristique de dilatation de l'élément dilatateur (14) permet une modification reproductible du volume de l'élément dilatateur au moyen de la pression pré-réglée.

6. Dispositif dilatateur selon l'une des revendications 1 à 4, caractérisé par le fait que la source de pression est conformée de telle manière que la pression à l'intérieur de l'élément dilatateur (14) s'élève d'abord lentement à chaque impulsion de pression jusqu'à ce que l'élément dilatateur (14) adhère à la paroi d'une cavité corporelle, ce qui est décelable en raison du fait que la pression diastolique présente dans un réservoir sous pression raccordé à la source de pression ne chute plus.

7. Dispositif dilatateur selon la revendication 6, caractérisé par le fait que la pression qui règne à l'intérieur du réservoir sous pression est utilisée pour définir la pression à pré-régler à l'intérieur de l'élément dilatateur (14) lors de la dilatation.

FIG.1

FIG.2